(19)

Europäisches Patentamt

European Patent Office

Office européen des brevets

(11) **EP 3 838 148 A1**

(12) **EUROPEAN PATENT APPLICATION**

(43) Date of publication:
**23.06.2021 Bulletin 2021/25**

(51) Int Cl.:
*A61B 5/282* (2021.01)   *A61B 5/361* (2021.01)
*A61B 5/00* (2006.01)   *A61B 5/36* (2021.01)
*A61B 5/363* (2021.01)

(21) Application number: **20213375.7**

(22) Date of filing: **11.12.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(30) Priority: **13.12.2019 IN 201921051884**

(71) Applicant: **Tata Consultancy Services Limited Maharashtra (IN)**

(72) Inventors:
- **JAYARAMAN, Srinivsan**
  **560066 Bangalore, Karnataka (IN)**
- **SHANMUKHAPP, Raghu Thopenahalli**
  **560066 Bangalore, Karnataka (IN)**
- **KULKARNI, Harshad**
  **560066 Bangalore, Karnataka (IN)**
- **SAHADEVAN, Joshin**
  **560066 Bangalore, Karnataka (IN)**
- **KADNI, Praveen Sureshrao**
  **560066 Bangalore, Karnataka (IN)**

(74) Representative: **Goddar, Heinz J.**
**Boehmert & Boehmert**
**Anwaltspartnerschaft mbB**
**Pettenkoferstrasse 22**
**80336 München (DE)**

(54) **METHOD AND DEVICE TO CONTINUOUSLY MONITOR AND DETERMINE CARDIAC HEALTH OF A PERSON**

(57)   Electrocardiogram (ECG) system has been adopted for almost a century to diagnose cardiovascular disease (CVD). Monitoring the cardiac signal provides an insight of CVD and function as an aiding tool for physician towards early detection of cardiac events. A method and wearable device to continuously monitor and determine the cardiac health of the person have been provided. The device is configured to monitor the cardiac system continuously in a partially or fully non-contact manner. The non-contact sensing is achieved by using a hybrid sensing technique. The device consists of a pair of electrodes, one electrode could be a contact sensor that will be touching the skin and the second sensor could be a non-contact sensor. The device facilitates to alert cardiac health monitoring locally or remote location. The device monitors cardiac health in the work environment rather than inducing stress among the participants by making them undergo a stress test.

FIG. 1

EP 3 838 148 A1

**Description**

CROSS-REFERENCE TO RELATED APPLICATIONS AND PRIORITY

The present application claims priority from Indian provisional specification no. 201921051884 filed on December 13, 2019.

TECHNICAL FIELD

The embodiments herein generally relate to the field of cardiac health monitoring. More particularly, but not specifically, the present disclosure provides a device and method to continuously monitor and determine the cardiac health of a person by capturing electrocardiogram (ECG).

BACKGROUND

Electrocardiogram (ECG) system has been adopted for almost a century to diagnose cardiovascular disease (CVD). Further, monitoring the cardiac signal continuous will provide an insight of CVD and function as an aiding tool for physician towards early detection of cardiac events. For an instant, monitoring the electrocardiogram (ECG) signal of an individual continuously during the day-to-day activities could detect the cardiac arrhythmia like AF during the occurrence. Currently, there is no such direct wrist-worn cardiac solution that could monitor and alert during movement, activity and, other day to day activities.

Currently used method to characterize an individual's cardiac disorder is established by collecting Electrocardiogram (ECG) in a controlled environment or conducting a voluntary study such as treadmill test, Holter system etc. Alternative methods were also adapted during an individual's movement by using PPG or voluntary contribute by touching a pair of electrodes attached either in wrist band like apple watch or phone case like AliveCore or other means in the wearable market. These means of acquiring ECG signal would either interpret or distract once activity and there is a high chance one could ignore its notification, alert, or time for contribution.

In general, none of the prior art have had addressed the acquiring, detection of a cardiac event in real-time or during the occurrence of cardiac disorder event using a wearable device. Besides, all the existing system exist require a voluntary contribution from the human to capture the ECG.

SUMMARY

The following presents a simplified summary of some embodiments of the disclosure to provide a basic understanding of the embodiments. This summary is not an extensive overview of the embodiments. It is not intended to identify key/critical elements of the embodiments or to delineate the scope of the embodiments. It's sole purpose is to present some embodiments in a simplified form as a prelude to the more detailed description that is presented below.

In view of the foregoing, an embodiment herein provides a wearable device for continuous monitoring of cardiac health of a person. The device comprises a first electrode, a second electrode, one or more hardware processors and a memory. The memory further comprises a classifier generation module, and the cardiac health monitoring module. The first electrode either of a contact type or a non-contact type of electrode. The second electrode of non-contact type of electrode, wherein the first electrode and the second electrode are configured to acquire an ECG signal. The classifier generation module further configured to generate a classifier and the classifier is pre-generated. The cardiac health monitoring module further configured to perform the steps of: capturing an ECG signal of the person using the wearable device, preprocessing the captured ECG signal of the person, extracting a plurality of test features from the preprocessed ECG signal, and detecting the presence of the cardiac disorder in the person using the plurality of test features and the classifier.

In another aspect, the embodiment here provides a method for continuous monitoring of cardiac health of a person. Initially, a wearable device is provided, the wearable device comprises a first electrode either of a contact type or a non-contact type and a second electrode of non-contact type, wherein the first electrode and the second electrode are configured to acquire an ECG signal, wherein the wearable device comprising a classifier and the classifier is pre-generated. Further, an ECG signal of the person is captured using the wearable device. In the next step the acquired ECG signal of the person is preprocessed. Further a plurality of test features are extracted from the preprocessed ECG signal. And finally, the presence of the cardiac disorder in the person is detected using the plurality of test features and the classifier.

In another aspect the embodiment here provides one or more non-transitory machine readable information storage mediums comprising one or more instructions which when executed by one or more hardware processors cause continuous monitoring of cardiac health of a person. Initially, a wearable device is provided, the wearable device comprises a first electrode either of a contact type or a non-contact type and a second electrode of non-contact type, wherein the first electrode and the second electrode are configured to acquire an ECG signal, wherein the wearable device comprising a classifier and the classifier is pre-generated. Further, an ECG signal of the person is captured using the wearable device. In the next step the acquired ECG signal of the person is preprocessed. Further a plurality of test features are extracted from the preprocessed ECG signal. And finally, the presence of the cardiac disorder in the person is detected using the plurality of test features and the classifier.

It should be appreciated by those skilled in the art that any block diagram herein represents conceptual views of illustrative systems embodying the principles of the present subject matter. Similarly, it will be appreciated that any flow charts, flow diagrams, state transition diagrams, pseudo code, and the like represent various processes which may be substantially represented in a computer-readable medium and so executed by a computing device or processor, whether or not such computing device or processor is explicitly shown.

BRIEF DESCRIPTION OF THE DRAWINGS

The accompanying drawings, which are incorporated in and constitute a part of this disclosure, illustrate exemplary embodiments and, together with the description, serve to explain the disclosed principles.

Fig. 1 shows a block diagram of a device for continuous monitoring of cardiac health of a person according to an embodiment of the present disclosure.

Fig. 2 shows the device for continuous monitoring of cardiac health of the person according to an embodiment of the present disclosure.

Fig. 3 shows the placement position of the device according to an embodiment of the disclosure.

Fig. 4 shows a functional flow diagram of the device for continuous monitoring of cardiac health of the person according to an embodiment of the present disclosure.

Fig. 5 shows a flowchart illustrating the steps involved in continuous monitoring of cardiac health of the person according to an embodiment of the present disclosure.

Fig. 6 illustrates a graphical representation of the slope of the ECG signal according to an embodiment of the present disclosure.

Fig. 7 illustrates a graphical representation of the features extracted using a slope-based method with marking on the signal plot according to an embodiment of the present disclosure.

DETAILED DESCRIPTION

Exemplary embodiments are described regarding the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the spirit and scope of the disclosed embodiments. It is intended that the following detailed description be considered as exemplary only, with the true scope and spirit being indicated by the following claims.

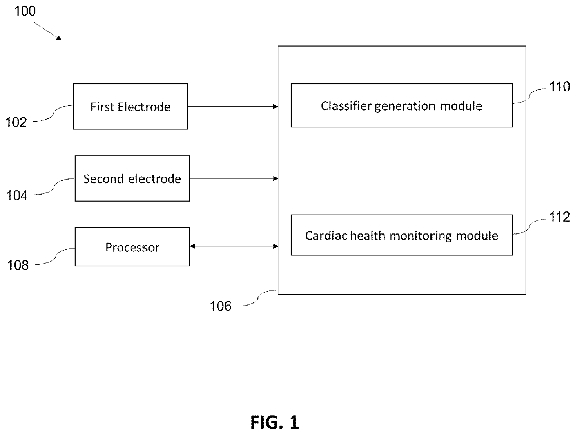

Referring now to the drawings, and more particularly to Fig. 1 through Fig. 7, where similar reference characters denote corresponding features consistently throughout the figures, there are shown preferred embodiments and these embodiments are described in the context of the following exemplary system and/or method.

According to an embodiment of the disclosure, a device 100 for continuous monitoring of cardiac health of a person is shown in the schematic overview of Fig. 1. The device 100 is a wearable device and can be worn by the person. The device 100 can be interchangeably referred as the wearable device 100 or the sensor device 100 in the disclosure. The wearable device 100 is also configured to transmit to the result of monitoring to a remote destination through wireless communication for medical assistance. The device 100 is adapted to acquire electrocardiogram (ECG) using non-contact (in a partially or fully) sensing mechanism. This non-contact sensing is achieved by using a hybrid sensing technique as explained in the later part of the disclosure. The device 100 is configured to monitor or determine the cardiac disorders like arrhythmia, atrial fibrillation, and so on, in the real-time rather than inducing stress among the participants by making them undergo a stress test or perform a relaxation exercise.

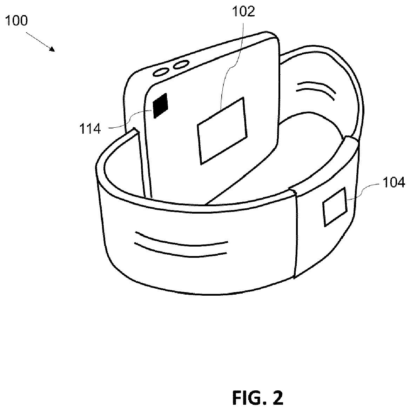

According to an embodiment of the disclosure, the device 100 comprises a first electrode 102, a second electrode 104, a memory 106 and one or more hardware processors 108 as shown in the block diagram of Fig. 2. The one or more hardware processors 108 work in communication with at least one memory 106. The one or more hardware processors 108 are configured to execute a plurality of algorithms stored in at least one memory 106. The memory 106 further includes a plurality of modules for performing various functions. The memory 106 includes a classifier generation module 110 and a cardiac health monitoring module 112. The memory 108 may further comprise other modules for performing certain functions.

According to an embodiment of the disclosure, the wearable device 100 utilizes a non-contact sensing mechanism. This could be achieved by using a hybrid sensing technique. Normally, the ECG requires a pair of electrode/sensor of which, one sensor could be in contact sensor say resistive sensor that will be touching the skin and second sensor could be a non-contact sensor say a capacitive based sensor. In an embodiment of the present disclosure, the first electrode 102 is either a contact type or a non-contact type of electrode. The second electrode 104 is a non-contact type of electrode. The first electrode 102 and the second electrode 104 are configured to acquire the ECG signal, wherein the ECG signal of an individual is acquired when the wearable sensor device comes to a predefined position on the body of the individual.

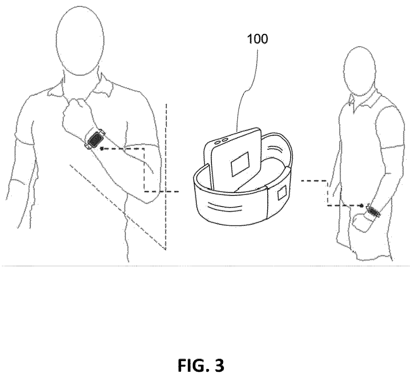

According to an embodiment of the disclosure, the wearable device 100 is worn on the wrist of the person as shown in Fig. 3. The wearable device 100 can come into two predefined positions when the ECG signal can be sensed as shown in Fig. 3. The first position is by bringing the wrist closer to the heart of the person and the second position is the by bringing the wrist closer to the thigh of the person. Both the position are such a way that the person can continue doing his normal work.

In another embodiment of the disclosure, the wearable device 100 can be worn on the neck of the person. The design of the device may vary depending on the place where the person is wearing the wearable device 100. In case of neck wearable device, the first and the second electrode are both the non-contact type of electrodes and can be worn as a necklace. In this case, the device 100 is configured to capture single-lead ECG configuration.

According to an embodiment of the disclosure, the pair of electrode is made of two different material and sensing are performed using two different phenomena. To be more specific, the contact type of electrode senses using skin resistance. The contact type of electrode may be made of sliver or sliver-sliver-chloride. The non-contact electrode will be using capacitive phenomena. The non-contact electrode may be made of copper, gold or platinum.

According to an embodiment of the disclosure, the memory 106 further comprises the classifier generation module 110 as shown in the block diagram of Fig. 1. The classifier generation module 110 is configured to generate a classifier. The classifier generation is a one-time process and it is generated pre-generated. There is no need to generate the classifier every time when we want to monitor the health of the person. The classifier is configured to classify the test sample received from the person as healthy or not healthy.

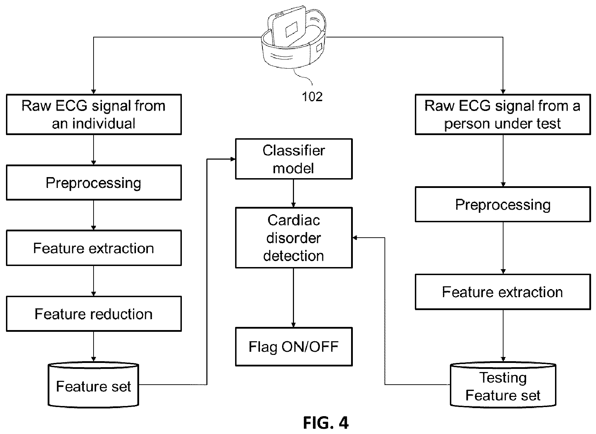

As shown in the flow diagram of Fig. 4, for generating the classifier ECG signal is acquired from a plurality of individuals with known cardiac health. The classifier generation module 110 is configured to preprocess the acquired ECG signal, extract a plurality of features from the preprocessed ECG signal. The plurality of features is then reduced based on the predefined criteria to get the feature set. The classifier generation module 110 further configured to generate the classifier using the plurality of features.

According to an embodiment of the disclosure, the plurality of features is extracted using the slope based feature extraction method. In this method, the slope of the ECG signal within a window size of 'n' number of samples is performed to extract the morphological details. The slope of the ECG signal has both positive and negative values due to increasing and decreasing peaks in an ECG waveform. The slope of the signal is calculated using Equation (1).

$$S_{slope}(i) = tan^{-1}(S(i+n) - S(i))/n \ldots\ldots\ldots\ldots (1)$$

where =1, 2...N-n,
S(i) = Extracted ECG Signal with samples 1 to N and n=Window size

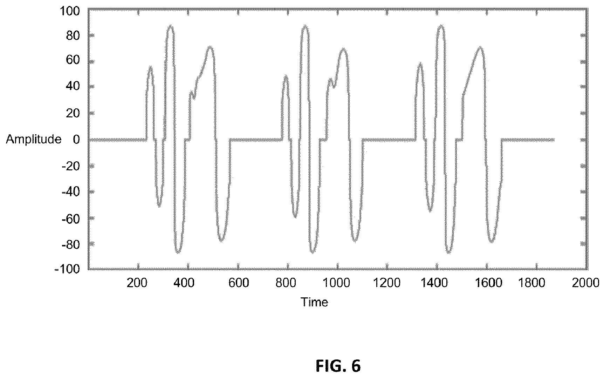

The window size depends on the number of samples between the Q peak and R peak in the ECG signal. A standard range of values is defined for the inclination angle of the P wave, QRS complex and T wave for both normal and abnormal ECG. Thus, from the defined range of slope values for the ECG waveform, the slope values between the minimum positive slope value and the maximum negative slope values are removed to eliminate any noise. For finding the window size, the R peak is found by differentiating the ECG signal and the Q wave is detected as the negative peak immediately before the detected R peak. The slope of the signal within this window is found for the entire signal as shown in Fig. 6.

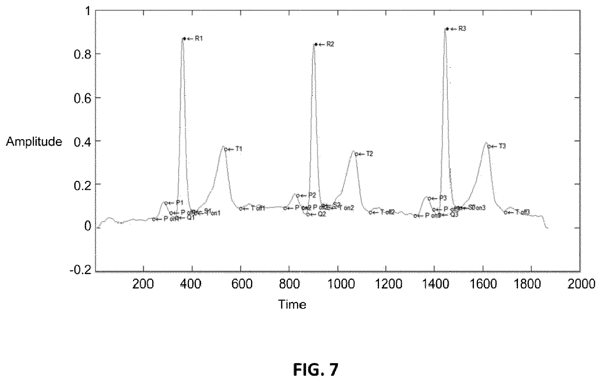

The first positive peak is the P_on, the first negative peak is P and the following zero crossings is P_off. Similarly, the procedure has been performed to identify the QRS complex and T wave. The features extracted using the slope method with a mark on the signal plot is shown in Fig. 7. Accuracy of 97.09% had been obtained for this method for a database of 25 patient's digital ECG records

According to an embodiment of the disclosure, the memory 106 further comprises the cardiac health monitoring module 112. The cardiac health monitoring module 112 is configured to acquire the ECG signal of the person captured using the wearable device 100, preprocess the acquired ECG signal of the person and extract a plurality of test features from the preprocessed ECG signal as shown in the flow diagram of Fig. 4. The cardiac health monitoring module 112 also configured to determine the cardiac health of the person using the plurality of test features and the classifier. The cardiac health monitoring module 112 determines or monitors various cardiac disorders like arrhythmia, atrial fibrillation, and so on, in the real-time (work environment) rather than inducing stress among the participants by making them undergo a stress test or perform a relaxation exercise.

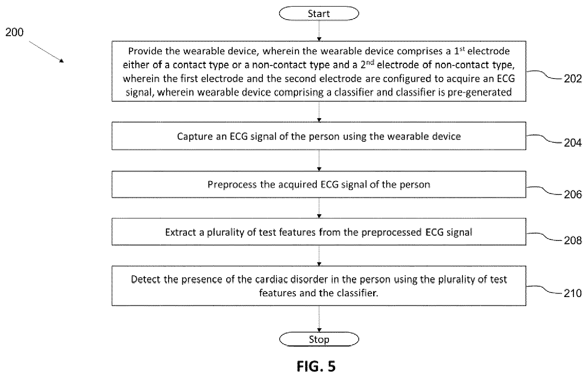

In operation, a flowchart 200 illustrating a method for continuous monitoring of cardiac health of the person as shown in FIG. 5. Initially, at step 202, the wearable device is provided. The device comprises the first electrode 102 either of a contact type or a non-contact type of electrode and the second electrode 104 of non-contact type of electrode, wherein the first electrode 102 and the second electrode 104 are configured to acquire the ECG signal. The wearable device 104 comprising a classifier and the classifier is pre-generated.

At step 204, the ECG signal of the person who is being monitored is captured using the wearable device 104. The ECG signal is captured in real-time continuously. At step 206, the acquired ECG signal of the person is preprocessed. At step 208, a plurality of test features is extracted from the preprocessed ECG signal. And finally at step 210, the presence of the cardiac disorder in the person detected using the plurality of test features and the generated classifier. It should be appreciated that the presence of cardiac disorder can be communicated to a distant location. So that a reactive measure can be taken to improve the cardiac health of the person.

According to an embodiment of the disclosure, the wearable device 100 further configured to perform auto impedance mismatching correction.

According to an embodiment of the disclosure, the wearable device 100 further comprises an accelerometer 114 to capture the movement of the individual as shown in Fig. 2. The captured movement then can further be used to remove the movement artifact from the captured ECG signal. Further, the accelerometer signal can be combined with the ECG signal using a decision fusion technique to improve the accuracy of cardiac health determination.

According to an embodiment of the disclosure, the plurality of features such as morphological features extracted was used to detect the arrhythmias. In an example, three different abnormalities have been considered, namely, Sinus bradycardia, Sinus tachycardia and premature ventricular contraction (PVC). Feature parameter were P wave duration, QRS complex durations, T wave duration, PR intervals, QT intervals, and ST intervals are chosen as feature vectors. In an example, two classifiers are used to detect arrhythmias, namely Dynamic time warping (DTW) and Adaboost classifier and their performance were compared. It should be appreciated that any such machine learning or classifier technique could be used by a person skilled in the art

**Dynamic Time Warping** (DTW)

The DTW classifier is based on the ranking of the prototypes by the distance to the query. Let, F = (f1.....fn) and G = (g1.....gm) be two time series of length n and m, respectively. To align the two sequences using DTW, an n-by-m matrix was constructed whose (i, j) th element is the Euclidean distance d (i, j) between two points $f_i$ and $g_j$. The (i, j) th matrix element corresponds to the alignment between the points $f_i$ and $g_j$. A warping path, R is a contiguous sets of matrix elements that defines a mapping between F and G and is written as R = {$r_1$.....$r_S$} where, max (m, n) < S < m + n - 1. The warping path is typically subject to several constraints such as boundary conditions, continuity, monotonicity, and windowing. The DTW algorithm finds the point-to-point correspondence between the curves, which satisfies the above constraints and yields the minimum sum of the costs associated with the matching of the data points. There are exponentially many warping paths that satisfy the above conditions. The path that minimizes the warping cost is shown in equation (2),

$$D(F, G) = \ min \sum_{s=0}^{s} r_s \ \dots\dots\dots\dots \ (2)$$

The warping path can be found efficiently using dynamic programming to evaluate a recurrence relation, which defines the cumulative distance $\gamma(i,j)$ up to the element (i, j) as the sum of d (i, j), the cost of dissimilarity between the $i^{th}$ and the $j^{th}$ points of the two sequences and the minimum of the cumulative distances up to the adjacent elements as shown in equation (3):

$$\gamma(i,j) = d(i,j) + \min\{\gamma(i-1,j), \gamma(i,j-i), \gamma(i-1,j-1)\} \ \dots\dots \ (3)$$

In an example, the classification procedure based on DTW yielded the following results.

Table 1- DTW classification results

| Types | Total No. Of Records | Classified | Misclassified |
|---|---|---|---|
| Normal | 25 | 24 | 1 |

(continued)

| Types | Total No. Of Records | Classified | Misclassified |
|---|---|---|---|
| Sinus Tachycardia | 8 | 8 | 0 |
| Sinus Bradycardia | 7 | 7 | 0 |
| Pvc | 5 | 5 | 0 |

**Adaboost Classifier:**

In this study, multiclass AdaBoost has been used in identifying arrhythmia detection. Adaboost classifier increases the accuracy of the weak classifier by reinforcing training on misclassified samples and assigns appropriate weights to each weak classifier. The final classification is given by equation (4)

$$h\left(x\right) \begin{cases} 1, if \sum_{i=1}^{t} \alpha_t h_t \geq threshold \\ 0, otherwise \end{cases} \quad \ldots\ldots \quad (4)$$

where 1 indicates that the sample has been correctly classified. In this experiment, stumps are used as a weak classifier. For reassigning the weights to the weak classifier 5000 iterations were performed and this was experimentally found to yield better results.

Because it may have potential advantages such as higher classification performance, more rapid recognition process time and extension of recognition features, Adaboost was applied for the detection of cardiac arrhythmia. Each class of ECG type i.e. normal or arrhythmic, a label +1 or -1 is assigned to it. A large number of weak classifiers around 5000 are chosen. Decision stumps are chosen for classification. Decision stumps make a prediction based on the value of just a single input feature. The input value if greater than the prediction value then the feature vector belongs to one class else it belongs to another class. Initially, a set of training vectors are fed for classification. Labels are assigned for each input. A set of testing vectors are given as inputs for classification. Based on the labels assigned to each of the testing vectors, the classification or misclassification is decided.

Table 2 - Adaboost classification results

| Types | Total No. Of Records | Classified | Misclassified |
|---|---|---|---|
| Normal | 25 | 24 | 1 |
| Sinus Tachycardia | 8 | 8 | 0 |
| Sinus Bradycardia | 7 | 7 | 0 |
| Pvc | 5 | 5 | 0 |

The Adaboost classifier is implemented and the classification results are as shown in Table 4. The sensitivity of the classifier is evaluated and the average sensitivity is found to be 99%. Table 3 presents the performance of the classification system for different arrhythmias. The performance of the detection of an arrhythmia is measured on the parameters of false rejection (FR), false acceptance (FA), false acceptance rate (FAR) and false rejection rate (FRR) reported by the system.

Table 3 Classification of ECG for different arrhythmias, Case -1 is normal, Case-2 is sinus bradycardia, Case -3 sinus tachycardia, and Case -4 is PVC

| | Precision (%) | Sensitivity (%) | Specificity (%) | Accuracy (%) |
|---|---|---|---|---|
| Case1 | 100 | 96 | 100 | 97.78 |
| Case2 | 87.5 | 100 | 97.37 | 97.78 |
| Case3 | 88.89 | 100 | 97.29 | 97.78 |
| Case4 | 83.33 | 100 | 97.5 | 97.78 |

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

The embodiments of the present disclosure herein solve the problems monitoring the health of the person without forcing him to do any of the activity. The disclosure provides a method and system for continuous monitoring of cardiac health of a person.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device. The hardware device can be any kind of device which can be programmed including e.g. any kind of computers like a server or a personal computer, or the like, or any combination thereof. The device may also include means which could be e.g. hardware means like e.g. an application-specific integrated circuit (ASIC), a field-programmable gate array (FPGA), or a combination of hardware and software means, e.g. an ASIC and an FPGA, or at least one microprocessor and at least one memory with software modules located therein. Thus, the means can include both hardware means and software means. The method embodiments described herein could be implemented in hardware and software. The device may also include software means. Alternatively, the embodiments may be implemented on different hardware devices, e.g. using a plurality of CPUs.

The embodiments herein can comprise hardware and software elements. The embodiments that are implemented in software include but are not limited to, firmware, resident software, microcode, etc. The functions performed by various modules described herein may be implemented in other modules or combinations of other modules. For the purposes of this description, a computer-usable or computer-readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope and spirit of the disclosed embodiments. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, non-volatile memory, hard drives, CD ROMs, DVDs, flash drives, disks, and any other known physical storage media.

It is intended that the disclosure and examples be considered as exemplary only, with a true scope of disclosed embodiments being indicated by the following claims.

## Claims

1. A processor-implemented method (200) for continuous monitoring of cardiac health of a person using a wearable device, the method comprising:

   providing the wearable device, wherein the wearable device comprises a first electrode either of a contact type or a non-contact type and a second electrode of non-contact type, wherein the first electrode and the second electrode are configured to acquire an ECG signal, wherein the wearable device comprising a classifier and the classifier is pre-generated (202);
   capturing an ECG signal of the person using the wearable device (204);

preprocessing, via one or more hardware processors, the acquired ECG signal of the person (206);

extracting, via one or more hardware processors, a plurality of test features from the preprocessed ECG signal (208); and

detecting, via one or more hardware processors, the presence of the cardiac disorder in the person using the plurality of test features and the classifier (210).

2. The method according to claim 1 further comprising the step of generating the classifier as follows:

acquiring the ECG signal of a plurality of individuals with known cardiac health using the wearable device, wherein the ECG signal is acquired when the wearable device comes to a predefined position on the body of the plurality of individuals,

preprocessing, via one or more hardware processors, the acquired ECG signal,

extracting, via one or more hardware processors, a plurality of features from the preprocessed ECG signal, and

generating, via one or more hardware processors, the classifier using the plurality of features;

3. The method according to claim 1, wherein the wearable sensor device is worn at one of the wrists, the neck of a person.

4. The method according to claim 3, wherein when the wearable sensor device is worn in the neck the device comprises two non-contact electrodes present near to the body.

5. The method according to claim 3, wherein when the wearable sensor device is worn in the wrist, the device is configured to capture more than one lead configuration sequentially.

6. The method according to claim 3, wherein when the wearable sensor device is worn in the neck, the device is configured to capture single-lead ECG configuration.

7. The method according to claim 1 wherein the cardiac disorder is at least one of an arrhythmia comprising atrial fibrillation, Bradycardia, and Tachycardia.

8. The method according to claim 1 wherein the classifier generated is one of a dynamic time warping or an Adaboost classifier.

9. The method according to claim 1, wherein the plurality of features are extracted using slope base feature extraction methodology.

10. The method according to claim 1, wherein the wearable device further configured to perform auto impedance mismatching correction.

11. The method according to claim 1 further comprising the step of combining the accelerometer with the ECG signal using a decision fusion technique to improve the accuracy of cardiac health determination.

12. A wearable device (100) for continuous monitoring of cardiac health of a person, the device comprising:

a first electrode (102) either of a contact type or a non-contact type of electrode;

a second electrode (104) of non-contact type of electrode, wherein the first electrode and the second electrode are configured to acquire an ECG signal;

one or more hardware processors (108);

a memory (106) in communication with the one or more hardware processors, the memory further comprising:

a classifier generation module (110), wherein the classifier generation module further configured to generate a classifier and the classifier is pre-generated; and

a cardiac health monitoring module (112), wherein the cardiac health monitoring module further configured to perform the steps of:

capturing an ECG signal of the person using the wearable device,

preprocessing the captured ECG signal of the person,

extracting a plurality of test features from the preprocessed ECG signal, and

detecting the presence of the cardiac disorder in the person using the plurality of test features and the classifier.

13. The wearable device according to claim 12 further comprises an accelerometer to capture the movement of the individual to remove the movement artifact.

14. The wearable device according to claim 12, wherein the first electrode or a contact electrode is a resistive electrode and made up of Silver - Silver chloride and the second electrode or a non-contact electrode is the capacitive electrode and made up of at least one of Copper, Platinum or Gold.

15. One or more non-transitory machine readable information storage mediums comprising one or more instructions which when executed by one or more hardware processors cause managing a plurality of events, the instructions cause:

providing the wearable device, wherein the wearable device comprises a first electrode either of a contact type or a non-contact type and a second electrode of non-contact type, wherein the first electrode and the second electrode are configured to acquire an ECG signal, wherein the wearable device comprising a classifier and the classifier is pre-generated;
capturing an ECG signal of the person using the wearable device;
preprocessing the acquired ECG signal of the person;
extracting a plurality of test features from the preprocessed ECG signal; and
detecting the presence of the cardiac disorder in the person using the plurality of test features and the classifier.

FIG. 1

**FIG. 2**

FIG. 3

Raw ECG signal from
an individual

↓

Preprocessing

↓

Feature extraction

↓

Feature reduction

↓

Feature set

102

Classifier
model

↓

Cardiac
disorder
detection

↓

Flag ON/OFF

Raw ECG signal from a
person under test

↓

Preprocessing

↓

Feature extraction

↓

Testing
Feature set

**FIG. 4**

200

```
                              ( Start )
                                  │
                                  ▼
┌─────────────────────────────────────────────────────────────────┐
│ Provide the wearable device, wherein the wearable device         │
│ comprises a 1st electrode either of a contact type or a          │
│ non-contact type and a 2nd electrode of non-contact type,        │──202
│ wherein the first electrode and the second electrode are         │
│ configured to acquire an ECG signal, wherein wearable device     │
│ comprising a classifier and classifier is pre-generated          │
└─────────────────────────────────────────────────────────────────┘
                                  │
                                  ▼
┌─────────────────────────────────────────────────────────────────┐
│ Capture an ECG signal of the person using the wearable device    │──204
└─────────────────────────────────────────────────────────────────┘
                                  │
                                  ▼
┌─────────────────────────────────────────────────────────────────┐
│ Preprocess the acquired ECG signal of the person                 │──206
└─────────────────────────────────────────────────────────────────┘
                                  │
                                  ▼
┌─────────────────────────────────────────────────────────────────┐
│ Extract a plurality of test features from the preprocessed       │──208
│ ECG signal                                                       │
└─────────────────────────────────────────────────────────────────┘
                                  │
                                  ▼
┌─────────────────────────────────────────────────────────────────┐
│ Detect the presence of the cardiac disorder in the person        │──210
│ using the plurality of test features and the classifier.         │
└─────────────────────────────────────────────────────────────────┘
                                  │
                                  ▼
                              ( Stop )
```

**FIG. 5**

**FIG. 6**

**FIG. 7**

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2019/358464 A1 (VOLOSIN KENT [US] ET AL) 28 November 2019 (2019-11-28) | 12,14,15 | INV. A61B5/282 |
| A | * paragraphs [0043], [0063], [0136], [0147], [0224], [0246] * <br> * figure 1 * | 1-11 | A61B5/361 <br> A61B5/00 <br> A61B5/36 <br> A61B5/363 |
| X | US 2019/275335 A1 (VOLPE SHANE S [US] ET AL) 12 September 2019 (2019-09-12) | 12,14,15 | |
| A | * paragraphs [0086], [0090], [0102], [0103], [0134] * <br> * figure 1 * | 1-11 | |
| X | US 2018/146922 A1 (WANG YUANXIANG [US] ET AL) 31 May 2018 (2018-05-31) | 12-15 | |
| A | * paragraphs [0024], [0032], [0071], [0076], [0091] * <br> * figure 3 * | 1-11 | |

TECHNICAL FIELDS
SEARCHED (IPC)

A61B

The present search report has been drawn up for all claims

| Place of search | Date of completion of the search | Examiner |
|---|---|---|
| The Hague | 18 May 2021 | Worms, Georg |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
.................................................................................
& : member of the same patent family, corresponding
document

EPO FORM 1503 03.82 (P04C01)

**ANNEX TO THE EUROPEAN SEARCH REPORT
ON EUROPEAN PATENT APPLICATION NO.**

EP 20 21 3375

This annex lists the patent family members relating to the patent documents cited in the above-mentioned European search report.
The members are as contained in the European Patent Office EDP file on
The European Patent Office is in no way liable for these particulars which are merely given for the purpose of information.

18-05-2021

| Patent document cited in search report | | Publication date | Patent family member(s) | | Publication date |
|---|---|---|---|---|---|
| US 2019358464 | A1 | 28-11-2019 | EP<br>US<br>WO | 3801237 A1<br>2019358464 A1<br>2019226581 A1 | 14-04-2021<br>28-11-2019<br>28-11-2019 |
| US 2019275335 | A1 | 12-09-2019 | NONE | | |
| US 2018146922 | A1 | 31-05-2018 | CN<br>US | 108113663 A<br>2018146922 A1 | 05-06-2018<br>31-05-2018 |

EPO FORM P0459

For more details about this annex : see Official Journal of the European Patent Office, No. 12/82

**EP 3 838 148 A1**

**Patent documents cited in the description**

- IN 201921051884